# EUROPEAN PATENT SPECIFICATION

(11) **EP 2 074 987 B1**
(45) Date of publication and mention of the grant of the patent: **26.06.2013**
(21) Application number: 08172770.3
(22) Date of filing: 23.12.2008
(51) Int. Cl.: A61K 9/127, A61K 38/12

(54) **Liposome composition of colistin for intravenous, oral, and cutaneous therapeutic use, and process for its preparation**
Liposomzusammensetzung von Colistin zur intravenösen, oralen und dermalen therapeutischen Verwendung und Verfahren zu deren Herstellung
Composition de liposomes de colistine pour une utilisation thérapeutique intraveineuse, orale et cutanée, et son procédé de préparation

(30) Priority: 28.12.2007 IT RM20070681
(43) Date of publication of application: 01.07.2009
(73) Proprietor: Tascini, Carlo, 55048 Torre del Lago-Viareggio (LU) (IT); Tascini, Giovanni, 00144 Rome (IT)
(72) Inventor: Tascini, Carlo, 55048 Torre del Lago-Viareggio (LU) (IT); Tascini, Giovanni, 00144 Rome (IT)
(74) Representative: Sarpi, Maurizio

(56) References cited:
- WO-A-2007/117550
- COLOME C ET AL: "Interaction of aminoglycosides and colistin with model membranes: Liposomes and monolayers" INTERNATIONAL JOURNAL OF PHARMACEUTICS, ELSEVIER BV, NL, vol. 90, no. 1, 10 February 1993 (1993-02-10), pages 59-71, XP023736569 ISSN: 0378-5173 [retrieved on 1993-02-10]
- MESTRES C ET AL: "Interaction of colistin with lipids in liposomes and monolayers" INTERNATIONAL JOURNAL OF PHARMACEUTICS (AMSTERDAM), vol. 160, no. 1, 12 January 1998 (1998-01-12), pages 99-107, XP002520424 ISSN: 0378-5173
- HANCOCK R E: "Peptide antibiotics" LANCET THE, LANCET LIMITED. LONDON, GB, vol. 349, no. 9049, 8 February 1997 (1997-02-08), pages 418-422, XP004839148 ISSN: 0140-6736
- JENSEN T ET AL: "Colistin inhalation therapy in cystic fibrosis patients with chronic Pseudomonas aeruginosa lung infection" JOURNAL OF ANTIMICROBIAL CHEMOTHERAPY, OXFORD UNIVERSITY PRESS, GB, vol. 19, no. 6, 1 January 1987 (1987-01-01), pages 831-838, XP002085835 ISSN: 0305-7453

## Description

### SUMMARY OF THE INVENTION

The present invention relates to a liposome composition containing the antibiotic colistin, which, thanks to the fact of being vehicled by liposomes, can be administered via intravenous route at higher dosages with greater clinical effectiveness, reaching higher tissue concentrations and without determining serious toxicity. In oral formulations, the liposome composition of colistin can be used as intestinal decontaminant, whereas, in topical formulations, such as creams or gels, it can be used in the treatment of skin infections or in the prophylaxis of infections in patients affected by burns. Also forming the subject of the present invention is the process for preparation of the aforesaid liposome composition containing colistin.

### STATE OF THE ART

### The antibiotic colistin

Colistin or polymyxin E is a polypeptide antibiotic, which was isolated in Japan in 1947 from *Bacillus Polymyxa* subsp. *Colistinus* and has been available for clinical use since 1959.

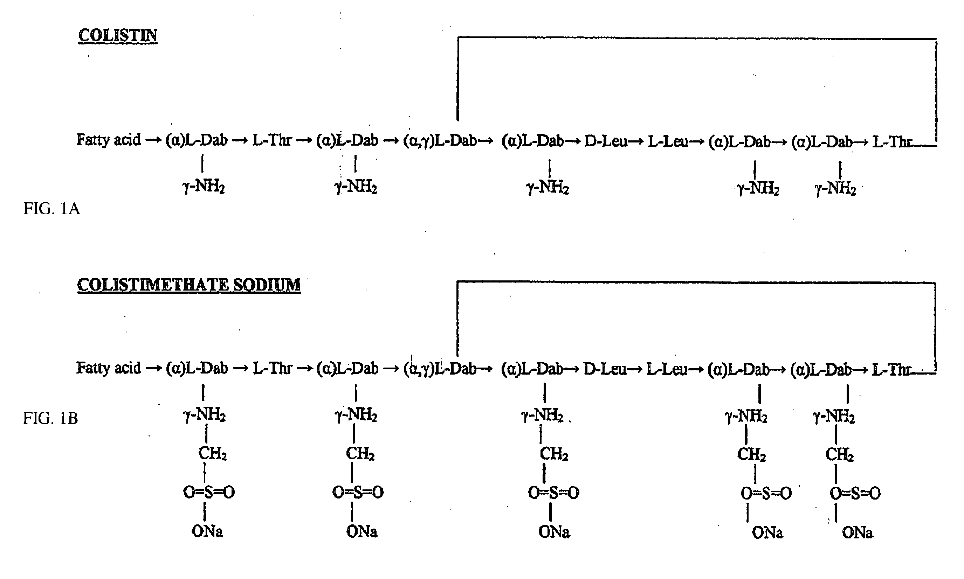

Colistin has a heptapeptide structure and an *N-*terminal side chain acetylated by a fatty acid to form decapeptides as shown in Figure 1A.

The ensemble of different decapeptides strictly correlated with one another forms colistin, the two major components of which are colistin A to colistin B, which differ from one another for their fatty acids: 6-methyl octanoic acid and 6-methyl heptanoic acid, respectively.

It is known that colistin is a bactericide against Gram-negative bacteria with a mechanism similar to that of detergents on the external membrane and on the cytoplasmatic membrane. It can in fact bind to the lipopolysaccharides and the phospholipids of the membrane and chelates also the cations thereof, thus causing damage to the membrane, from which there derives the osmotic lysis of the micro-organism.

Colistin administered by intravenous route is generally in its methane-sulphonate form, which, however, proves 4 to 8 times less active than colistin but in any case presents bactericidal activity.

WO 2007/117550 discloses a liposomal formulation of antiinfectives like colistin.

### The world clinical context

Currently, in hospital environments throughout the world, infections resulting from Gram-negative bacteria have been found that prove resistant to chemiotherapy.

The most dangerous of these bacteria is *Pseudomonas aeruginosa MDR* (Multi Drug Resistant).

*P. aeruginosa* is intrinsically resistant to many antibiotics including the beta-lactamics, the old quinolones, chloramphenicol (CAF), tetracyclines, macrolides, sulphamides, and rifampicin, even though rifampicin can have a synergistic effect if associated to the polymyxins.

Furthermore, a reduced permeability of the external membrane and the presence of numerous efflux pumps enable resistance of *P. aeruginosa* to numerous antibiotics (quinolones, tetracyclines, CAF, macrolides, sulphamides, aminoglycosides, and many beta-lactamics). If to these mechanisms there are associated the numerous beta-lactamases, then the therapeutic tools against these bacteria are reduced to just a few molecules.

"Pan-resistant" strains are more and more frequently reported, even though the sensitivity to polymyxin is rarely reported.

Consequently, colistin has re-acquired considerable importance on account of the development of strains that are resistant to all the antibiotics, except polymyxin.

In this connection, colistin has proven to be a drug that may be associated to different molecules with a quite a good effectiveness even though it presents serious renal side effects that limit use thereof.

Colistin can be associated also to beta-lactamic antibiotics, in particular carbapenemics, having mechanisms of resistance on the outer wall of the micro-organism (this is the target of colistin that acts therein as tensioactive agent).

The pharmacokinetics of colistin is affected by its hydrophilia, which does not allow sufficient concentrations to be reached in certain tissues; in fact, it does not penetrate very far into the lung, into the bone, or into the CNS.

### Description of the invention

The description of the invention will be more readily understandable with reference to the annexed plates of drawings, in which :
Figure 1A shows the chemical structure of colistin, where α and y indicate -NH₂ in the peptide bond, Dab is diaminobutyric acid, Leu is leucine, and Thr is threonine;
Figure 1B shows the chemical structure of a derivative thereof, such as sodium colistimethate;
Figure 2 is a block diagram of the process of preparation of liposome colistin according to the invention.

By the term "colistin" in the present invention are meant all the salified forms and/or the known derivatives of the antibiotic colistin, such as sodium colistimethate illustrated in Figure 1B.

It is known that the colloidal carriers, such as liposomes are able to modify the pharmacokinetics of the drugs and their toxicity. In general, the association to the liposomes does not lead to any loss of the antimicrobial capacity.

According to the present invention, it is envisaged to vehicle colistin through the liposomes to bring it into high concentrations in the tissue sites of infection without having any side effects at a renal level.

Liposomes are hollow microspheres formed by one or more double lipid layers. Ever since the seventies they have been used - in experimental form - as vehicles of drugs, and understanding of their behaviour *in vivo* has enabled targeted studies to be conducted on the specific treatment of given pathological conditions. Currently, several pharmaceutical firms involved in biotechnology work exclusively with liposomes for the development of different treatments: antibiotic treatments, antitumoral treatments, allergic-sensitization treatments, gene therapy, etc.

The interest of liposomes regards their membrane (constituted by colesterol and phospholipids, such as phosphatidyl coline and diacetylphosphate), the structure, composition, and proportions of which are practically identical to those of the membrane of the host cells.

Phospholipids are esters of glycerol with fatty acids in positions 1 and 2 and with phosphoric acid in position 3. The latter is in turn bound to amine bases of low molecular weight. They are fundamental components of the cell membranes and of the lipoprotein complexes involved in the absorption and transport of lipids and have a hydrophobic tail and a hydrophilic head which, by dissolving in water, reorganize themselves in the following way: the hydrophobic tails attract one another, whereas the hydrophilic heads set themselves in contact with the outside and with the internal aqueous environment (the arrangement is similar to that of micelles). As a result, double lipid layers are formed, which close to form small vescicles similar to the cells of the organism and to its organelles.

The aforesaid spheres or liposomes constitute small deposits that can contain an antigen, an antibiotic, an allergen, a drug or a gene (gene therapy) and be introduced into the organism without causing immune reactions of rejection.

The characteristics of liposomes can be modified according to the different drugs vehicled. For example, in order to reduce the rate of degradation of the liposome and slow down release of its contents, the composition and dimensions thereof are modified. It is also possible to increase the affinity of liposomes for a given tissue by modifying their composition, the electrical charge (by adding stearylamine, vescicules with positive charge are obtained; with diacetyl phosphate, negative charges are obtained) or also by adding receptors or adhesive antigens, which increases the concentration of the drug in the target organ.

Given that liposomes can encapsulate drugs, proteins and enzymes, the systems can be administered by intravenous, oral, or intramuscular route. On the other hand, liposomes present various disadvantages as vectors for release of drugs in so far as they are subject to oxidative degradation and must be conserved and manipulated in a nitrogen atmosphere.

The antibiotic colistin (or polymyxin E) can be chemiadsorbed on a substrate of liposomes, in an autoclave. The liposome composition that is obtained forms the subject of the present invention and can be prepared in the form of powder for being administered, after prior appropriate reconstitution, intravenously to the patient, or else in the form of syrup for oral use or in the form of an ointment for dermatological use.

It has surprisingly been discovered that the liposome substrate of colistin, by reducing the renal contact of the antibiotic, can prevent the associated side effects, in particular renal toxicity, maintaining its antibacterial characteristics intact.

The liposome composition according to the present invention can obviously be comprised in appropriate pharmaceutical compositions for clinical use, having, for example, the technical form of powder to be reconstituted for intravenous use, syrups for oral use, or creams for dermatological use, according to modes of production known to the person skilled in the sector.

In particular, the liposome composition forming the subject of the invention can be used for preparation of pharmaceutical compositions for clinical treatment against multiresistant Gram-negative germs both in intravenous formulation and in oral and topical formulation. Creams or gels with a base of the liposome composition of colistin have proven extremely effective in the clinical treatment of skin infections and in the prophylaxis of infections in patients affected by burns. The process of preparation

Also forming the subject of the present invention is the process of preparation of liposome colistin.

The process that is described is of a discontinuous (batch-wise) type. The individual operations used are, in order:
I) Dosage
ii) Mixing
iii) Distillation
iv) Drying
v) Homogenization (by means of stirring)
vi) Chemiadsorption (by means of cooling and forced centrifugation)

In all of the processing steps it is necessary to blow with nitrogen to prevent degradation of the phospholipids.

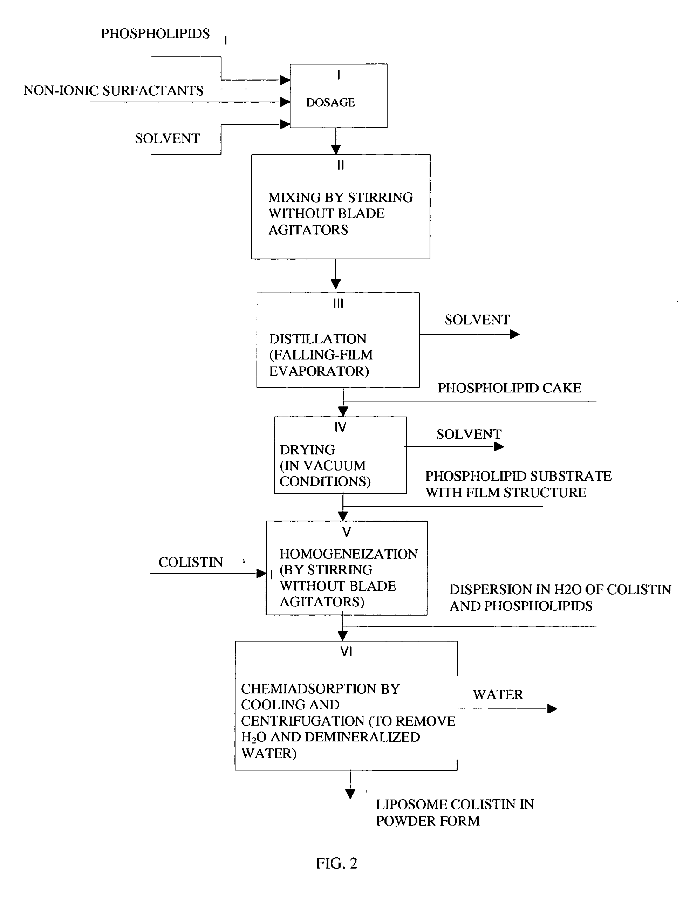

Provided by way of non-limiting example is a detailed description of the process of preparation, on the basis of the block diagram of Figure 2. Parameters and compositions may be appropriately modified.
I) In a reactor with hemispherical bottom are dosed in order: solvent (constituted by a mixture of chloroform and ethanol, respectively 70 wt% and 30 wt%) in an amount 20 times in excess with respect to the phospholipids to be used, phospholipids (dimyristoyl phosphatidylglycerol), with a weight ratio of 5:1 with respect to the colistin to be produced, with the addition of non-ionic surfactants as required.
ii) The above solution is mixed by stirring, but without the use of blade agitators; duration of mixing: at least 2 hours.
iii) Next, the solvent is distilled in a falling-film rotary evaporator. The maximum limit of temperature that can be tolerated by the product, which is of 40°C, must be observed.
iv) The cake thus obtained must be dried of the residual traces of solvent by means of distillation in vacuum conditions. In this step, the maximum limit of temperature of 35°C must be observed. The time of this operation is approximately 2-3 hours, which can be optimized. There is thus obtained the substrate of phospholipids with film structure.
v) At this point, the physiological solution of colistin is added, with a concentration of 10 mg/ml of demineralized water. The product is homogenized by means of stirring, without use of blades, for a sufficient time to disperse the solute completely (typically 1 h at 150 r.p.m.). The dispersion that is thus obtained has a typical composition by weight of:
   colistin 1/phospholipids 5/water 100.
vi) In order to obtain chemiadsorption of colistin on the liposome substrate recourse is had to cooling, at a minimum temperature of 4°C, and at the same time the water is eliminated by means of forced centrifugation with a centripetal acceleration equal to at least 20,000 g, for a sufficient time to expel all the water (typically 90-120 minutes). The final product is extracted by centrifugation: colistin on liposome substrate in the form of powder.

## Claims

1. A process for preparation of a liposome composition containing colistin for intravenous, oral and topical therapeutic use, **characterized in that** it envisages the following steps:
I) a dosage step, in which an appropriate amount of phospholipids is dissolved in a mixture of chloroform and ethanol, in a round-bottom reactor;
II) a mixing step, in which the solution obtained is mixed by stirring;
III) a distillation step, in which said solution is processed in a rotary evaporator, distilling the solvent to obtain a cake of phospholipids;
IV) a drying step, in which the cake thus obtained is dried of the residual traces of solvent by means of distillation in vacuum conditions at a temperature not higher than 35°C to obtain a substrate of phospholipids with film structure;
V) a homogenization step, in which added to said substrate of phospholipids is a physiological solution of colistin, to obtain a dispersion that is homogenized by stirring for a sufficient time to disperse the solute completely;
VI) a step of chemiadsorption of colistin on the liposome substrate, in which the homogenized dispersion is cooled to a minimum temperature of 4°C and finally centrifuged with an acceleration of at least 20,000 g, for a sufficient time to expel all the water, so as to extract from the centrifuge said colistin on liposome substrate in the form of powder.

2. The process for preparation of a liposome composition according to Claim 1, **characterized in that** the phospholipids that are dissolved in the mixture of chloroform and ethanol have a weight ratio of 5:1 with respect to colistin to be produced and **in that** added to said phospholipids are non-ionic surfactants as required.

3. The process for preparation of a liposome composition according to Claim 1, **characterized in that** in the dosage step, the amount of solvent, constituted by the mixture of chloroform and ethanol, is at least 20 times in excess with respect to the phospholipids to be used.

4. The process for preparation of a liposome composition according to Claim 1, **characterized in that** the duration of the mixing step is at least 2 hours.

5. The process for preparation of a liposome composition according to Claim 1, in which the evaporator used in the distillation step is a falling-film rotary evaporator and the maximum operating temperature is kept below 40°C.

6. The process for preparation of a liposome composition according to Claim 1, in which the drying step has a duration of approximately 2-3 hours.

7. The process for preparation of a liposome composition according to Claim 1, in which the physiological solution of colistin that is added in the homogenization step has a concentration of 10 mg/ml of demineralized water.

8. Use of a liposome composition obtained by the process according to claim 1 for preparation of pharmaceutical compositions in the clinical treatment against Gram-negative MDR bacteria, in intravenous formulation.

9. Use of a liposome composition obtained by the process according to claim 1, for preparation of pharmaceutical compositions in the clinical treatment against Gram-negative MDR bacteria, in oral formulation.

10. Use of a liposome composition obtained by the process according to claim 1, for preparation of pharmaceutical compositions in the clinical treatment against Gram-negative MDR bacteria, in topical formulations.

11. Liposome composition obtained by the process according to claim 1, for preparation of creams or gels that can be used in clinical treatment for skin infections.

12. Liposome composition obtained by the process according to claim 1, for preparation of creams or gels that can be used in the prophylaxis of infections in patients affected by burns.

## Patentansprüche

1. Verfahren zur Herstellung einer Colistin enthaltenden Liposomverbindung zur intravenösen, oralen und lokalen therapeutischen Verwendung, **dadurch gekennzeichnet, dass** es die folgenden Schritte beabsichtigt:
I) einen Dosierungsschritt, bei dem eine entsprechende Menge von Phospholipiden in einer Mischung aus Chloroform und Äthanol in einem Rundbodenreaktor aufgelöst wird;
II) einen Mischungsschritt, bei dem die erhaltene Lösung durch Umrühren gemischt wird;
III) einen Destillationsschritt, bei dem die Lösung in einem Rotationsverdampfer bearbeitet wird, indem das Lösungsmittel destilliert wird, um einen Filterkuchen aus Phospholipiden zu erhalten;
IV) einen Trocknungsschritt, bei dem der so erhaltene Filterkuchen von den übrig gebliebenen Spuren von Lösungsmittel mittels Destillation unter Vakuumbedingungen bei einer Temperatur getrocknet wird, die nicht höher als 35°C ist, um ein Substrat aus Phospholipiden mit hauchdünnem Überzug zu erhalten;
V) einen Homogenisierungsschritt, bei dem eine physiologische Lösung aus Colistin zu dem Substrat aus Phospholipiden hinzugefügt wird, um eine Dispersion zu erhalten, die durch Umrühren eine ausreichende Zeit lang homogenisiert wird, um den gelösten Stoff vollständig zu dispergieren;
VI) einen Schritt der Chemiadsorption von Colistin auf dem Liposomsubstrat, bei dem die homogenisierte Dispersion auf eine Mindesttemperatur von 4°C gekühlt und schließlich mit einer Beschleunigung von mindestens 20 000g eine ausreichende Zeit lang zentrifugiert wird, um das gesamte Wasser herauszupressen, damit das Colistin auf Liposomsubstrat in Form von Pulver extrahiert wird.

2. Verfahren zur Herstellung einer Liposomverbindung nach Anspruch 1, **dadurch gekennzeichnet, dass** die Phospholipide, die in der Mischung aus Chloroform und Äthanol aufgelöst sind, ein Gewichtsverhältnis von 5 : 1 in Bezug auf herzustellendes Colistin besitzen, und dadurch, dass bei Bedarf zu den Phospholipiden nicht ionische oberflächenaktive Substanzen hinzugefügt werden.

3. Verfahren zur Herstellung einer Liposomverbindung nach Anspruch 1, **dadurch gekennzeichnet, dass** in dem Dosierungsschritt die Menge von durch die Mischung von Chloroform und Äthanol gebildetem Lösungsmittel mindestens mehr als das 20-fache bezüglich der zu verwendenden Phospholipide beträgt.

4. Verfahren zur Herstellung einer Liposomverbindung nach Anspruch 1, **dadurch gekennzeichnet, dass** die Dauer des Mischungsschrittes mindestens 2 Stunden beträgt.

5. Verfahren zur Herstellung einer Liposomverbindung nach Anspruch 1, bei dem der im Destillationsschritt verwendete Verdampfer ein Fallfilmrotationsverdampfer ist und die maximale Betriebstemperatur unter 40°C gehalten wird.

6. Verfahren zur Herstellung einer Liposomverbindung nach Anspruch 1, bei dem der Trocknungsschritt eine Dauer von ungefähr 2 bis 3 Stunden aufweist.

7. Verfahren zur Herstellung einer Liposomverbindung nach Anspruch 1, bei dem die physiologische Colistinlösung, die in dem Homogenisierungsschritt hinzugefügt wird, eine Konzentration von 10 mg/ml demineralisierten Wassers besitzt.

8. Verwendung einer durch das Verfahren nach Anspruch 1 erhaltenen Liposomverbindung zur Herstellung von Pharmaverbindungen bei der klinischen Behandlung gegen Gram-negative MDR-Bakterien (Bakterien mit Arzneimittelmehrfachresistenz) in intravenöser Zubereitung.

9. Verwendung einer durch das Verfahren nach Anspruch 1 erhaltenen Liposomverbindung zur Herstellung von Pharmaverbindungen bei der klinischen Behandlung gegen Gram-negative MDR-Bakterien, in oraler Zubereitung.

10. Verwendung einer durch das Verfahren nach Anspruch 1 erhaltenen Liposomverbindung zur Herstellung von Pharmaverbindungen bei der klinischen Behandlung gegen Gram-negative MDR-Bakterien, in lokalen Zubereitungen.

11. Liposomverbindung, die durch das Verfahren nach Anspruch 1 erhalten wird, zur Herstellung von Cremes oder Gels, die bei klinischer Behandlung für Hautinfektionen verwendet werden können.

12. Liposomverbindung, die durch das Verfahren nach Anspruch 1 erhalten wird, zur Herstellung von Cremes oder Gels, die in der Prophylaxe von durch Verbrennungen verursachten Infektionen bei Patienten verwendet werden können.

## Revendications

1. Procédé de préparation d'une composition de liposomes contenant de la colistine pour utilisation thérapeutique intraveineuse, orale et topique, **caractérisé en ce qu'**il comprend les étapes suivantes :
I) une étape de dosage, dans laquelle une quantité appropriée de phospholipides est dissoute dans un mélange de chloroforme et d'éthanol, dans un réacteur à fond rond ;
II) une étape de mélange, dans laquelle la solution obtenue est mélangée par agitation ;
III) une étape de distillation, dans laquelle ladite solution est traitée dans un évaporateur rotatif, en distillant le solvant pour obtenir une galette de phospholipides ;
IV) une étape de séchage, dans laquelle la galette ainsi obtenue est séchée des traces résiduelles de solvant au moyen d'une distillation en conditions de vide à une température non supérieure à 35°C pour obtenir un substrat de phospholipides avec structure de film ;
V) une étape d'homogénéisation, dans laquelle une solution physiologique de colistine est ajoutée audit substrat de phospholipides, pour obtenir une dispersion qui est homogénéisée par agitation pendant un temps suffisant pour disperser complètement le soluté ;
VI) une étape d'absorption chimique de colistine sur le substrat de liposomes, dans laquelle la dispersion homogénéisée est refroidie à une température minimale de 4°C et finalement centrifugée avec une accélération d'au moins 20.000 g, pendant un temps suffisant pour expulser toute l'eau, de manière à extraire de la centrifugeuse ladite colistine sur substrat de liposomes sous la forme de poudre.

2. Procédé de préparation d'une composition de liposomes selon la revendication 1, **caractérisé en ce que** les phospholipides qui sont dissouts dans le mélange de chloroforme et d'éthanol ont un rapport de poids de 5:1 par rapport à la colistine à produire et **en ce que** des agents de surface non ioniques sont ajoutés auxdits phospholipides comme nécessaire.

3. Procédé de préparation d'une composition de liposomes selon la revendication 1, **caractérisé en ce que** dans l'étape de dosage, la quantité de solvant, constitué par le mélange de chloroforme et d'éthanol, est au moins 20 fois en excès par rapport aux phospholipides à utiliser.

4. Procédé de préparation d'une composition de liposomes selon la revendication 1, **caractérisé en ce que** la durée de l'étape de mélange est d'au moins 2 heures.

5. Procédé de préparation d'une composition de liposomes selon la revendication 1, dans lequel l'évaporateur utilisé dans l'étape de distillation est un évaporateur rotatif à film tombant et la température de fonctionnement maximale est maintenue au-dessous de 40°C.

6. Procédé de préparation d'une composition de liposomes selon la revendication 1, dans lequel l'étape de séchage a une durée d'environ 2-3 heures.

7. Procédé de préparation d'une composition de liposomes selon la revendication 1, dans lequel la solution physiologique de colistine qui est ajoutée dans l'étape d'homogénéisation a une concentration de 10 mg/ml d'eau déminéralisée.

8. Utilisation d'une composition de liposomes obtenue par le procédé selon la revendication 1, pour la préparation de compositions pharmaceutiques dans le traitement clinique contre des bactéries multi-résistantes gram-négatives, en formulation intraveineuse.

9. Utilisation d'une composition de liposomes obtenue par le procédé selon la revendication 1, pour la préparation de compositions pharmaceutiques dans le traitement clinique contre des bactéries multi-résistantes gram-négatives, en formulation orale.

10. Utilisation d'une composition de liposomes obtenue par le procédé selon la revendication 1, pour la préparation de compositions pharmaceutiques dans le traitement clinique contre des bactéries multi-résistantes gram-négatives, dans des formulations topiques.

11. Composition de liposomes obtenue par le procédé selon la revendication 1, pour la préparation de crèmes ou gels qui peuvent être utilisés dans un traitement clinique pour des infections cutanées.

12. Composition de liposomes obtenue par le procédé selon la revendication 1, pour la préparation de crèmes ou gels qui peuvent être utilisés dans la prophylaxie d'infections chez des patients affectés par des brûlures.
